# EUROPEAN PATENT APPLICATION

(11) **EP 4 595 961 A1**
(43) Date of publication of application: **06.08.2025**
(21) Application number: 24305179.4
(22) Date of filing: 01.02.2024
(51) Int. Cl.: A61K 31/397, A61K 31/4965, A61K 45/06, A61P 9/10

(54) **SELEXIPAG FOR USE IN THE TREATMENT AND/OR PREVENTION OF ATHEROSCLEROSIS**

(71) Applicant: SORBONNE UNIVERSITE, 75006 Paris (FR); Assistance Publique - Hôpitaux de Paris, 75012 Paris (FR); Institut National de la Santé et de la Recherche Médicale, 75013 Paris (FR); Centre National de la Recherche Scientifique, 75016 Paris (FR)
(72) Inventor: GLORIAN, Martine, 75013 PARIS (FR); LEGUEUX CAJGFINGER, Yohan, 75005 PARIS (FR)
(74) Representative: Cabinet Beau de Loménie

(57) **Abstract**

The present invention relates to the use of Selexipag to treat and prevent atherosclerosis, and resulting atherosclerotic cardiovascular diseases. In some aspects, the Selexipag prevents plaque formation, inhibits plaque development, reduces such plaque, reduces lipid core, prevents plaque rupture, decreases the susceptibility of plaque to rupture, and prevent thrombus associated with such plaque rupture. In some aspects, the Selexipag prevents smooth muscle cells from internalising cholesterol, and stimulates cholesterol efflux from the arterial wall.

## Description

### Field of the invention

The present invention relates to Selexipag for use in the treatment and/or the prevention of atherosclerosis in a subject, whereby Selexipag is used to prevent atherosclerotic cardiovascular disease resulting from atherosclerosis in said subject.

### Background of the invention

Atherosclerosis is the most prevalent and damaging health challenge facing societies around the world. Atherosclerosis is a progressive, chronic and inflammatory disease affecting large and medium-sized arteries in the human body, and which may lead to the death of the subject.

Atherosclerosis involves the development of plaques within the arterial walls, wherein plaques are atherosclerotic plaques. Plaques are made up among others of cholesterol, cellular debris, and inflammatory cells. Such plaques may cause the affected artery to narrow, leading to a reduced blood flow that may lead to an insufficient blood supply to organs irrigated by the artery.

Plaques comprise a lipid core and a fibrous capsule, wherein the lipid core is usually called lipid-rich core. An increase of the lipid core leads to the thinning of the fibrous capsule, making plaques susceptible to rupture. Plaque rupture may lead to the formation of a thrombus blocking the affected artery.

Atherosclerosis-related events described here above may lead to an atherosclerotic cardiovascular disease (ASCVD), which corresponds to a clinical consequence of atherosclerosis. ASCVD includes coronary artery disease (CAD), cerebrovascular disease (CVD), and peripheral artery disease (PAD). Clinical symptoms of CAD include, among others, stable angina, unstable angina, myocardial infarction, and sudden cardiac death. Clinical symptoms of CVD include, among others, ischemic stroke, haemorrhagic stroke or mini-stroke. Clinical symptoms of PAD include, among others, infections and tissue death.

Myocardial infarction and ischemic stroke are the most life-threatening complications of CAD. Especially, myocardial infarctions represent 85% of CAD. CAD represents the first cause of mortality worldwide.

Majority of these acute events are the consequences of intraluminal thrombosis triggered by atherosclerotic plaque rupture.

Several risk factors for atherosclerosis and resulting ASCVD are disclosed in the literature, such as hypercholesterolemia, hypertension, diabetes, plasma LDL and HDL cholesterol levels. Indeed, a robust inverse relationship has been demonstrated between HDL-cholesterol level and cardiovascular events, independently of LDL-cholesterol level. One of the main functions of HDL is to promote cholesterol efflux (CE) from peripheral tissues and atherosclerotic plaques and to transport it to the liver where it will be converted into bile acids excreted in faeces as bile salts. This mechanism is known as reverse cholesterol transport. This function is the most widely accepted mechanism for HDL protection effect. An increased cholesterol efflux capacity (CEC) is associated with decreased cardiovascular events [1] [2].

Knowledge of this mechanism has led to the development of HDL mimetics as a potential therapeutic tool [3]. The most promising HDL mimetic treatment is CSL112, an infusible, plasma-derived apoA-I combined with phosphatidyl choline. CSL112 allows a rapid and significant enhancement of CEC and shows anti-atherosclerotic properties *in vitro* and *in vivo* [4]. Notably, when infused to hypercholesterolemic mice, CSL112 allows a significantly decrease in lipid content and CD68 cells (macrophages-derived or vascular smooth muscle cells-derived foam cells) in advanced atherosclerotic plaques [5]. CSL112 is currently in phase 3 of clinical development to evaluate its effectiveness in reducing cardiovascular events. [6]

Several medicaments are known for treating and/or preventing atherosclerosis, and therefore for preventing resulting ASCVD. The following list of medicaments is not exhaustive:
- Low-Density Lipoprotein-cholesterol (LDL-C) lowering drugs such as statins, PCSK9 inhibitors, ezetimibe, cholestyramine, lomitapide, fibrates, ethyl esters of omega-3 acids,
- antiplatelet and anticoagulant agents,
- anti-inflammatory agents, and
- blood pressure medications.

### Statins

Statins are the most commonly prescribed drugs for diseases associated with high cholesterol levels. They work by inhibiting an enzyme called HMG-CoA reductase, inducing the reduction of the cholesterol production in the liver and increasing the removal of low-density lipoprotein cholesterol (LDL-C) from the bloodstream.

Statins used to treat or prevent atherosclerosis, and therefore to prevent resulting atherosclerotic cardiovascular diseases, include, among others, atorvastatin, fluvastatin, pravastatin, simvastatin, and rosuvastatin.

However, statins are not effective in all patients. Indeed, they only reduce cardiovascular events by about 30% in high-cardiovascular risk patients. In addition, statins may cause intolerance in some patients, with adverse side effects in up to 10% of patients mainly linked to a nocebo effect. Statin adverse side effects include, among others, muscle pain, joint pain, gastrointestinal issues, liver issues, diabetes and probably brain memory issues.

### PCSK9 inhibitors

PCSK9 refers to Proprotein convertase subtilisin/kexin type 9. The effect of PCSK9 is a decrease in the number of LDL-C receptors. PCSK9 inhibitors work by inhibiting the circulating PCSK9, thereby increasing LDL-C receptor levels, which promotes the metabolism of LDL-C by the liver and the removal of LDL-C from the bloodstream. PCSK9 inhibitors used to treat and/or prevent atherosclerosis, and therefore to prevent resulting atherosclerotic cardiovascular diseases, include evolocumab, alirocumab and inclisiran, which have the drawback of being expensive. PCSK9 inhibitors are most often considered when patients have persistent high LDL-C despite maximal tolerated statin dose. Like statins, they reduce cardiovascular events by about 30% in high-cardiovascular risk patients.

### Ezetimibe

Ezetimibe works by inhibiting the intestinal absorption of cholesterol.

### Cholestyramine

Cholestyramine is a resin which works by trapping bile acids and reducing the intestinal absorption of cholesterol.

### Lomitapide

Lomitapide works by inhibiting a protein which transfers triglycerides, leading to a decrease of triglycerides and cholesterol levels in the bloodstream.

### Ethyl esters of omega-3 acids

Ethyl esters of omega-3 acids constitute essential fatty acids which decrease blood triglyceride levels in the bloodstream and reduce the susceptibility of blood to clotting.

### Antiplatelet agents

Antiplatelet agents work by inhibiting platelet activation, reducing blood clot formation, and then lowering the risk of cardiovascular events. Antiplatelet medications used to treat or prevent atherosclerosis, and therefore to prevent resulting atherosclerotic cardiovascular diseases, include, among others, aspirin, clopidrogel, prasugrel, and ticagrelor.

### Anticoagulant agents

Anticoagulant agents, also known as blood thinners, are not used as a direct treatment for atherosclerosis or atherosclerotic cardiovascular diseases itself, but they may play a role in managing certain complications and reducing the risk of cardiovascular events associated with atherosclerotic cardiovascular diseases, such as thrombosis, embolisms or ischemic events. Anticoagulants used to treat or prevent atherosclerosis, and therefore to prevent resulting atherosclerotic cardiovascular diseases, include, among others, acenocoumarol, apixaban, dabigatran etexilate, dalteparin sodium, enoxaparin sodium, fondaparinux sodium, calcium heparin, heparin sodium, nadroparin calcium, rivaroxaban, tinzaparin sodium, and warfarin sodium.

### Anti-inflammatory agents

Anti-inflammatory agents are not used as a direct treatment for atherosclerosis or atherosclerotic cardiovascular diseases itself, but they may play a role in managing the condition and reducing its progression by addressing the inflammatory component of the disease. Anti-inflammatory agents work by reducing inflammation, stabilizing atherosclerotic plaques, and modulating immune responses. Anti-inflammatory agents used to treat or prevent atherosclerosis, and therefore to prevent resulting atherosclerotic cardiovascular diseases include, among others, colchicine and nonsteroidal anti-inflammatory drugs (NSAIDs).

### Blood pressure medications

Blood pressure medications are not used as direct treatment for atherosclerosis or atherosclerotic cardiovascular diseases itself, but they may play a role in managing the condition and its associated risk factors. Blood pressure medications work by reducing arterial wall stress, and protecting the arterial endothelium. Blood pressure medications used to treat or prevent atherosclerosis, and therefore to prevent resulting atherosclerotic cardiovascular diseases, include, among others, diuretics, Angiotensin-Converting Enzyme (ACE) inhibitors, Angiotensin II Receptor Blockers (ARBs), alpha-blockers, beta-blockers, and calcium channel blockers.

### Fibrates

Fibrates are not first-line treatments for atherosclerosis or atherosclerotic cardiovascular diseases, but they may play a role in managing specific lipid abnormalities associated with atherosclerosis and ASCVD. They work by reducing triglyceride levels, increasing high-density lipoprotein cholesterol (HDL-C) levels, and inducing an anti-inflammatory effect. Fibrates used to treat or prevent atherosclerosis, and therefore to prevent resulting atherosclerotic cardiovascular diseases, include, among others, bezafibrate, ciprofibrate, fenofibrate, and gemfibrozil.

The treatments and preventions described here above may be used alone or in combination with a maximum tolerated statin dose. Such a combination can allow a slight improvement of the statin-based therapy.

In spite of a large number of medicaments used for treating and/or preventing atherosclerosis, there is still a need for developing new therapies that are safe and efficient for treating and/or preventing atherosclerosis, and that act directly on the main cause of the disease, i.e. the atherosclerotic plaque, thereby preventing the occurrence of ASCVD.

### Summary of the invention

In this regard, the present invention provides a new therapy for treating and/or preventing atherosclerosis in a subject, thereby preventing the occurrence of atherosclerotic cardiovascular diseases in said subject.

The present invention relates to Selexipag for use in the treatment and/or the prevention of atherosclerosis in a subject.

The details of one or more embodiments of the present invention are set forth in the description below. Other features, objects and advantages of the invention will be apparent from the description.

### Brief description of the figures

[Fig. 1] Acquisition of the foam phenotype from vascular smooth muscle cells. Ox-LDL: oxidized-LDL; PDGF: 10 ng/mL PDGF-BB (Platelet-Derived Growth Factor-BB).
[Fig. 2] Acquisition of the foam phenotype from vascular smooth muscle cells (n=4 to 6). Ctle: control; PDGF: 10 ng/mL PDGF-BB (Platelet-Derived Growth Factor-BB); ox-LDL: 75 µg/mL oxidized-LDL; L+P: oxLDL + PDGF-BB.
[Fig. 3] Effect of MRE-269 on cholesterol efflux in vascular smooth muscle cells-derived foam cells (n=6). Ctle: control; MRE: MRE-269; HDL: reconstituted HDL.
[Fig. 4] Effect of Selexipag on the internalisation of oxidized LDL by human aortic smooth muscle cells. PDGF-BB: 10ng/mL Platelet-Derived Growth Factor-BB; DiI-ox-LDL: 75 µg/mL Oxidized LDL labelled with DiI: 10 µg/mL.
[Fig. 5] Effect of Selexipag on the internalisation of oxidized LDL by human aortic smooth muscle cells (n=2). NT: control; DiI-ox-LDL: Oxidized LDL labelled with DiI; ACT: ACT-333679; PDGF-BB: 10ng/ml Platelet-Derived Growth Factor-BB.
[Fig. 6] Effect of Selexipag on the expression of genes ABCA1 and ABCG1 in human aortic smooth muscle cells (n=1 or 2). NT: control; ACT: ACT-333679; PDGF-BB: 10ng/mL Platelet-Derived Growth Factor-BB.

### Detailed description of the invention

### Definitions

The definitions and explanations below are for the terms as used throughout the entire application, including both the specification and the claims.

When describing the present invention, the terms used are to be construed in accordance with the following definitions, unless indicated otherwise.

The term "administration" or "administering" refers to providing the active ingredient, alone or as part of a pharmaceutically acceptable composition, to the patient in whom the disease, condition or symptom is to be treated or prevented.

The term "pharmaceutically acceptable" means approved by a federal or state regulatory agency or listed in the American or European pharmacopoeia, or in another generally recognised pharmacopoeia, for use in animals and humans.

A "pharmaceutical composition" means a composition comprising a pharmaceutically acceptable carrier. For example, a pharmaceutically acceptable carrier may be a diluent, adjuvant, excipient or vehicle with which the therapeutic agent is administered. These vehicles may be sterile liquids, such as water and oils, including those of petroleum, animal, vegetable or synthetic origin, such as peanut oil, soya oil, mineral oil, sesame oil, etc.. Water is a preferred vehicle when the pharmaceutical composition is administered intravenously. Saline solutions and aqueous solutions of dextrose and glycerol can also be used as liquid vehicles, particularly for injectable solutions. Pharmaceutically acceptable excipients include starch, glucose, lactose, sucrose, sodium stearate, glycerol monostearate, talc, sodium chloride, skimmed milk powder, glycerol, propylene glycol, water, ethanol and the like. When the pharmaceutical composition is suitable for oral administration, the tablets or capsules may be prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulphate). Tablets can be coated by processes well known in the state of the art. Liquid preparations for oral administration may take the form, for example, of solutions, syrups or suspensions, or may be presented as a dry product to be reconstituted with water or another suitable vehicle before use. Such liquid preparations can be prepared by conventional means with pharmaceutically acceptable vehicles such as suspending agents (e.g. sorbitol syrup, cellulose derivatives or hydrogenated edible fats); emulsifying agents (e.g. lecithin or acacia); non-aqueous carriers (e.g. almond oil, oily esters, ethyl alcohol or fractionated vegetable oils); and preservatives (e.g. methyl or propyl p-hydroxybenzoates or sorbic acid). Pharmaceutical compositions may also contain buffer salts, flavourings, colourings and sweeteners, as appropriate. The composition according to the invention is preferably a pharmaceutical composition.

The term "carrier", "excipient", or "vehicle" refer to an inert medium or carrier used as a solvent or diluent in which the active ingredient is formulated and/or administered, and which does not produce an adverse, allergic or other reaction when administered to an animal, preferably a human being. This includes all solvents, dispersion media, coatings, antibacterial and antifungal agents, isotonic agents, absorption retardants and other similar ingredients. For human administration, preparations must meet standards of sterility, general safety and purity as required by regulatory agencies such as, but without being limited to, the U.S. Food and Drug Administration (FDA) or European Medicines Agency (EMA). For the purposes of the invention, "pharmaceutically acceptable excipient" includes all pharmaceutically acceptable excipients as well as all pharmaceutically acceptable carriers, diluents, and/or adjuvants.

The term "Selexipag", also known as "NS-304", "ACT-293987", or "JNJ-678896049" refers to the molecule 2-{4-[(5,6-diphenylpyrazin-2-yl)(isopropyl)amino]butoxy}-*N-*(methylsulfonyl)acetamide of formula I, having the CAS number 475086-01-2. For example, Selexipag is commercialy available as Uptravi^{®}.

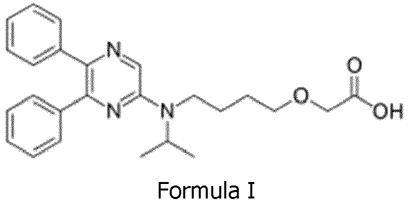

The term "Selexipag" also includes amorphous or crystalline forms of Selexipag, such as polymorphs thereof. The term "Selexipag" also includes anhydrous or hydrates thereof. The term "Selexipag" also includes solvates thereof. Such solvates include a molecule of a solvent bound through intermolecular forces or chemical bonds to one or more locations of the Selexipag molecule. Geometrical isomers (Z form and E form) of Selexipag or mixtures thereof are also contemplated. The term "Selexipag" also includes pharmaceutically acceptable salts thereof, which may readily be selected by those skilled in the art.

The term "pharmaceutically acceptable salt" refers to a salt that is non-toxic, biologically tolerable, or otherwise biologically suitable for administration to a subject. For example, Selexipag can be used in the form of a free base or acid, but can also be used after forming into a pharmaceutically acceptable salt by a known method. When the Selexipag is basic, examples of "salt" include salts of inorganic acids such as hydrochloric acid, sulfuric acid, nitric acid, phosphoric acid, hydrofluoric acid and hydrobromic acid, and salts of organic acids such as acetic acid, tartaric acid, lactic acid, citric acid, fumaric acid, maleic acid, succinic acid, methanesulfonic acid, ethanesulfonic acid, benzenesulfonic acid, p-toluenesulfonic acid, naphthalenesulfonic acid and camphorsulfonic acid. When the Selexipag is acidic, examples of "salt" include alkali metal salts such as sodium salt and potassium salt, and alkali earth metal salts such as calcium salt.

The term "MRE-269", also known as "ACT-333679", refers to the active metabolite of Selexipag, having the CAS number 475085-57-5. MRE-269 contributes to the therapeutic effects of Selexipag.

The term "treatment", "treat" or "treating" refers to a therapeutically administration aimed at reversing, alleviating, inhibiting or preventing the progression of the disease or condition to which such term is applied, or of one or more symptoms of such condition or disease.

The term "prevention", "prevent" or "preventing" means a prophylactic administration in a subject to prevent the occurrence of the disease or condition to which such term is applied, or of one more symptoms of such condition or disease.

The term "subject" or "patient" means any mammal that has or is at risk of developing a target disease or condition, and for whom treatment and/or prevention is desired. "Mammal" refers to any animal classified as a mammal, including humans, domestic and farm animals, such as dogs, horses, cats, cows, sheep, goats, pigs, etc. Preferably, the mammal is human.

The term "atherosclerosis" refers to a chronic pathologic condition resulting from the build-up of fats, cholesterol and other substances in and on the artery walls. This build-up is called atherosclerotic plaque. The plaque can cause arteries to narrow and stiffen, reducing blood flow. Plaque rupture can also occur, leading to a blood clot. Risk factors for atherosclerosis include, for example, diabetes, hypertension, dyslipidemia, and/or obesity.

The term "atherosclerotic cardiovascular disease", also known as "ASCVD", means a group of diseases that are caused by atherosclerosis and that can affect different locations throughout the body. ASCVD mainly results from the atherosclerotic plaques itself and/or from their rupture. Examples of ASCVD include coronary artery disease (CAD), cerebrovascular disease (CVD), or peripheral artery disease (PAD).

The term "coronary artery disease" or "CAD", also known as "coronary heart disease" or "CHD", "ischemic heart disease" or "IHD", or "myocardial ischemia", is a disease involving atherosclerotic plaques inside the coronary arterial walls, thus narrowing coronary arteries. This leads to, among others, poor blood flow to the coronary arteries, causing insufficient blood supply to the heart. Plaque rupture leads to a thrombus susceptible to totally obstruct the artery. Clinical symptoms of CAD include, among others, stable angina, unstable angina, myocardial infarction, and sudden cardiac death. Atherosclerosis is the underlying cause of most CAD.

The term "cerebrovascular disease" or "CVD" refer to a disease involving atherosclerotic plaques in carotid arterial walls. This leads to poor flow in cerebral arteries, causing insufficient blood supply to the brain. Clinical symptoms of CVD include, among others, ischemic stroke, haemorrhagic stroke or mini-stroke. Atherosclerosis is the underlying cause of most CVD.

The term "peripheral artery disease" or "PAD" is also known as "peripheral artery occlusive disease" or "PAOD", "peripheral obliterative arteriopathy" or "POA", or "peripheral vascular disease" or "PVD". PAD is a disease involving atherosclerotic plaques in peripheral arterial walls, thus narrowing peripheral arteries. This leads to poor blood flow to the peripheral arteries, causing insufficient blood supply to organs other than heart or brain, such as, among others, legs, arms, neck, and kidneys, more commonly in legs. Clinical symptoms of PAD include, among others, infection or tissue death. Atherosclerosis is the underlying cause of most PAD.

The term "plaque" refers to atherosclerotic plaque. Plaques are characterized by a build-up of fats comprising a lipid core and a fibrous capsule. The lipid core is usually called "lipid-rich core".

The term "foam cell" refers to a type of cell containing an accumulation of cholesterol, said cholesterol is mainly accumulated in the form of low-density lipoproteins (LDL) and/or cholesterol esters. Foam cells compose the lipid-rich core of atherosclerotic plaques. Foam cell formation is triggered by a number of factors including the uncontrolled uptake of modified LDL, the upregulation of cholesterol esterification and the impairment of mechanisms associated with cholesterol release. Modified LDL results essentially from oxidation and/or aggregation of LDL. Such accumulation results from uncontrolled internalisation of cholesterol in a cell and/or insufficient cholesterol efflux. Foam cells are derived from macrophages or vascular smooth muscle cells (VSMCs). The term "foam cell" includes formed foam cell and foam cell in development. In the context of atherosclerosis, foam cells secrete proteases involved in atherosclerotic plaque rupture.

The term "cholesterol efflux" refers to the process by which cholesterol is removed from cells, in particular towards High-Density Lipoproteins (HDL-mediated cholesterol efflux), and transported to the bloodstream where it can be addressed to the liver to be metabolized. In the context of atherosclerosis, an efficient cholesterol efflux may inhibit foam cell development, and/or prevent the formation of foam cells. A disrupted cholesterol efflux may lead to cholesterol accumulation in cells. In the context of atherosclerosis, a disrupted cholesterol efflux may lead to foam cells formation and/or foam cells development, and thus atherosclerosis progression.

The "cholesterol efflux capacity" or "CEC" is the ability of HDL to promote cholesterol efflux from cells, such as from foam cells. *In vitro* assays that measure the ability of individual HDLs to promote cholesterol efflux from macrophages-derived foam cells are commonly disclosed in the art [4] [7]. *In vitro* assays that measure cholesterol efflux from VSMC-derived foam cells are commonly disclosed in the art [8] [9] and in Example 1 herein.

The term "diabetes" refers to a chronic metabolic condition characterized by an elevated level of glucose in the bloodstream, resulting from defects in insulin production or action. This term includes, among others, type 1 diabetes, type 2 diabetes, gestational diabetes, monogenic diabetes, secondary diabetes, and all other forms of diabetes.

The term "hypertension", also known as "high blood pressure" refers to a chronic medical condition characterized by an elevated blood pressure level. This term includes, for example, primary (essential) hypertension, and secondary hypertension.

The term "dyslipidemia" or "dyslipidaemia" refer to a chronic medical condition characterized by an elevated blood level of lipids (i.e. cholesterol and triglycerides) in the bloodstream. Dyslipidemia can be characterized by an elevated VLDL-C level, an elevated LDL-C level and/or a low HDL-C level.

The term "obesity" refers to a chronic medical condition characterized by an elevated high body mass index (BMI). This term includes class I obesity (BMI from 30 to 34.99), class II obesity (BMI from 35 to 39.99) and class III obesity (BMI equal or higher to 40).

The term "SCORE" and "SCORE2" mean Systematic COronary Risk Evaluation and Systematic COronary Risk Evaluation 2, respectively. SCORE and SCORE2 refer to a 10-year cardiovascular risk assessment in a subject aged between 40 and 69 years old. The assessment includes at least the following parameters: the systolic blood pressure, the total cholesterol, HDL-C and LDL-C blood levels, the smoking status, the diabetes status, the age, the sex and the medical history of the subject. The result of this assessment may be classified as follows: low risk, moderate risk, high risk, and very high risk.

The term "SCORE-OP" or "SCORE2-OP" mean Systematic Coronary Risk Evaluation-Older Persons and Systematic Coronary Risk Evaluation 2-Older Persons, respectively. SCORE-OP or SCORE2-OP refers to a 10-year cardiovascular risk assessment in a subject aged 70 years or over. The assessment usually includes the following parameters: the systolic blood pressure, the total cholesterol, HDL-C and LDL-C blood levels, the smoking status, the diabetes status, the age, the sex and the medical history of the subject. The result of this assessment may be classified as follows: low risk, moderate risk, high risk, and very high risk.

The term "Framingham risk SCORE", "Framingham test", "Framingham risk assessment", "Framingham cardiovascular risk score", "Framingham risk prediction tool", or "Framingham 10-year risk score" refer to a 10-year cardiovascular risk assessment. The assessment usually includes the following parameters: the systolic blood pressure, the total cholesterol, HDL-C and LDL-C blood levels, the smoking status, the diabetes status, the age, the sex and the medical history of the subject. The result of this assessment may be classified as follows: low risk, moderate risk, high risk, and very high risk.

The term "ASCVD risk calculator", "atherosclerotic cardiovascular disease risk calculator", or "atherosclerotic cardiovascular disease risk estimator", refers to a 10-year cardiovascular risk assessment. The assessment usually includes the following parameters: the systolic blood pressure, the total cholesterol, HDL-C and LDL-C blood levels, the smoking status, the diabetes status, the race, the age, the sex and the medical history of the subject. The result of this assessment may be classified as follows: low risk, moderate risk, high risk, and very high risk.

The term "Globorisk SCORE" or "Globorisk cardiovascular risk prediction model" refer to a 10-year cardiovascular disease assessment. The assessment usually include the following parameters: the systolic blood pressure, the total cholesterol, HDL-C and LDL-C blood levels, the smoking status, the diabetes status, the region, the age, the sex and the medical history of the subject. The result of this assessment may be classified as follows: low risk, moderate risk, high risk, and very high risk.

The term "QRISK" and "QRISK2" mean "Quality risk" and "Quality risk 2", respectively. QRISK and QRISK2refer to a 10-year cardiovascular disease assessment. The assessment usually includes the following parameters: the systolic blood pressure, the total cholesterol, HDL-C and LDL-C blood levels, the smoking status, the diabetes status, the body mass index, the arterial fibrillation, the age, the ethnicity, the sex and the medical history of the subject. The result of this assessment may be classified as follows: low risk, moderate risk, high risk, and very high risk.

The term "Reynolds risk score" refer to a 10-year cardiovascular disease assessment. The assessment may include the following parameters: the C-reactive protein, triglyceride, total cholesterol, HDL-C and LDL-C blood levels, the smoking status, the diabetes status, the body mass index, the age, the sex and the medical history of the subject. The result of this assessment may be classified as follows: low risk, moderate risk, high risk, and very high risk.

The term "AHA/ACC ASCVD risk estimator plus" means "American Heart Association and the American College of Cardiology Atherosclerotic cardiovascular disease risk estimator plus" and refers to a 10-year cardiovascular disease assessment. The assessment usually include the following parameters: the systolic blood pressure, the total cholesterol, HDL-C and LDL-C blood levels, the smoking status, the diabetes status, the race, the age, the sex and the medical history of the subject. The result of this assessment may be classified as follows: low risk, moderate risk, high risk, and very high risk.

The term "per day" refers to the total dose administered to a subject in a day. The term "per day" may include one or more doses administered in a day, such as a daily dose, a twice daily dose, a thrice daily dose, or a forty daily dose.

An "effective dose" is a dose sufficient to effect beneficial or desired clinical results. An effective dose can be administered in one or more administrations. For purposes of this invention, an effective dose of Selexipag is a dose that is sufficient to palliate, ameliorate, stabilize, reverse, prevent, slow or delay the progression of the disease state, e.g. atherosclerosis or atherosclerotic plaque. Similar effects may be obtained with indicia appropriate for human patients, including without limitation C-reactive protein [CRP] and fibrinogen; lipoprotein-associated phospholipase A2 [Lp-PLA2] and myeloperoxidase [MPO]; growth differentiation factor-15 [GDF-15]) inflammatory markers; ambulatory arterial stiffness, IVUS imaging, and the like.

### Second medical use

The present invention provides a new therapy for treating and/or preventing atherosclerosis in a subject, thereby preventing the occurrence of atherosclerotic cardiovascular disease in said subject.

The Selexipag is approved for treating and/or preventing pulmonary arterial hypertension (PAH), and further developed for treating Buerger disease and Raynaud's phenomenon in systemic sclerosis.

The Applicants have shown that Selexipag can be used for treating atherosclerosis, which has not been disclosed or suggested in the prior art.

Therefore, the present invention relates to Selexipag for use in the treatment and/or the prevention of atherosclerosis in a subject.

The present disclosure also contemplates Selexipag active metabolite, namely MRE-269 (Cas n°475085-57-5), for use in the treatment and/or the prevention of atherosclerosis in a subject. Therefore, the term "Selexipag" may be replaced by MRE-269 in the present description to contemplate said disclosure.

As described herein above, atherosclerosis may lead to atherosclerotic cardiovascular disease (ASCVD). Thus, the treatment and/or prevention of atherosclerosis lead to the prevention of ASCVD. In some embodiments, the Selexipag is therefore used to prevent atherosclerotic cardiovascular disease (ASCVD).

ASCVD can be divided into three groups of diseases, depending on which arteries are affected. A first group, named coronary artery disease (CAD), concerns coronary arteries. A second group, named cerebrovascular disease (CVD), concerns carotid arteries. A third group, named peripheral artery disease (PAD), concerns peripheral arteries.

In some embodiments, atherosclerotic cardiovascular disease (ASCVD) is one or more disease selected from the group consisting of coronary artery disease (CAD), cerebrovascular disease (CVD) and peripheral artery disease (PAD).

In atherosclerosis, plaque formation and development lead to narrowed arteries. Plaque rupture can also occur, which may lead to a thrombus formation. Data from clinical trials indicate that it is the susceptibility of atherosclerotic plaque to rupture (vulnerable plaque), rather than its volume, that is the primary determinant of severe events of ASCVD. The characteristics of a plaque that is vulnerable to rupture include a thin fibrous cap separating the circulation from procoagulants in the plaque's lipid core; increased numbers of inflammatory cells (e.g., macrophages and T cells); and a relative paucity of vascular smooth muscle cells (VSMCs) in the fibrous capsule. Plaque stability reflects various dynamic factors: interaction of inflammatory cells, VSMCs production of the extracellular matrix that is the bulwark of the fibrous cap, inhibition of this process by certain cytokines, and increased degradation of the matrix by matrix metalloproteinases secreted by the lipid core.

The Applicants' results demonstrate or suggest that:
- Selexipag acts directly on the structure of the plaque itself;
- Selexipag inhibits atherosclerotic plaque lipid core formation and/or development;
- Selexipag reduces the atherosclerotic plaque lipid core content;
- Selexipag improves HDL-mediated cholesterol efflux, especially HDL-mediated cholesterol efflux from vascular smooth muscle cells-derived foam cells;
- Selexipag reduces internalisation of low-density lipoprotein (LDL);
- Selexipag improves plaque stability.

In some embodiments, the Selexipag is used to prevent atherosclerotic plaque formation, to inhibit atherosclerotic plaque development and/or to reduce atherosclerotic plaque.

Atherosclerotic plaque development can be measured in a subject using different techniques, such as using a cardiac CT angiography (CCTA) [10].

In some embodiments, the Selexipag is used to reduce atherosclerotic plaque lipid core. Reduction of atherosclerotic plaque lipid core improves plaque stabilization. Atherosclerotic plaque lipid core can be measured in a subject using different techniques, such as using magnetic resonance T₂ mapping [11] or by ultrasound [12] [13]. For example, reduction of atherosclerotic plaque lipid core may be measured in a subject by comparing the results of a magnetic resonance T₂ mapping or ultrasound before and after a treatment with Selexipag. For example, reduction of atherosclerotic plaque lipid core in the subject treated with Selexipag may be at least 10% compared to the atherosclerotic plaque lipid core of said subject before treatment with the Selexipag, such as by at least 15%, at least 20%, at least 25%, at least 30%, at least 35%, at least 40%, at least 45%, at least 50%. [14]

Prevention of atherosclerotic plaque formation, inhibition of atherosclerotic plaque development and/or stabilization of atherosclerotic plaques reduces the risk of plaque rupture, and consequently reduces the risk of developing an ASCVD.

In some embodiments, the Selexipag is used to prevent atherosclerotic plaque rupture.

In some embodiments, the Selexipag is used to decrease the susceptibility of atherosclerotic plaque to rupture.

In some embodiments, the Selexipag is used to prevent thrombus associated with atherosclerotic plaque rupture.

In some embodiments, the Selexipag is used to inhibit vascular smooth muscle cells (VSMCs) from internalising low-density lipoprotein-cholesterol (LDL-C).

In some embodiments, the Selexipag is used to stimulate (i.e. to increase) cholesterol efflux, such as HDL-mediated cholesterol efflux, such as cholesterol efflux from the arterial wall, such as HDL-mediated cholesterol efflux from the arterial wall, such as cholesterol efflux from foam cells, such as HDL-mediated cholesterol efflux from foam cells. The cholesterol efflux, as disclosed herein, in the subject treated with the Selexipag may be increased by at least 10% compared to the cholesterol efflux of said subject before treatment with the Selexipag, such as by at least 15%, at least 20%, at least 25%, at least 30%, at least 35%, at least 40%, at least 45%, at least 50%. The cholesterol efflux may be indirectly assessed by measuring the quantity of bile acids and/or sterols excreted in faeces, for example as disclosed in Eriksson et al. [15].

In some embodiments, the subject has diabetes, hypertension, dyslipidemia and/or obesity. The risk of developing atherosclerosis, and/or ASCVD, is increased if the subject has diabetes, hypertension, dyslipidemia and/or obesity.

In some embodiments, the subject has or had a smoking profile. The risk of developing atherosclerosis, and/or ASCVD, is increased if the subject has or had a smoking profile.

In some embodiments, the subject has a high or very high risk of developing atherosclerotic cardiovascular disease, said risk can be assessed by the Systematic COronary Risk Evaluation (SCORE), the Systematic COronary Risk Evaluation 2 (SCORE2), the Systematic COronary Risk Evaluation-Older Persons (SCORE-OP) the Systematic COronary Risk Evaluation 2-Older Persons (SCORE2-OP), the Framingham risk SCORE, the Globorisk SCORE, the QRISK, the QRISK2, the Reynolds risk score, or the AHA/ACC ASCVD risk estimator plus.

SCORE, SCORE2, SCORE2-OP, Framingham risk score, Globorisk score, QRISK, QRISK2, Reynolds risk score, and AHA/ACC ASCVD risk estimator plus, refer to cardiovascular risk assessments in a subject. The results of these risk assessments may be classified as follows: low risk, moderate risk, high risk and very high risk.

The Selexipag may be administered in a subject by a number of routes as determined by those skilled in the art. Preferably, the Selexipag is administered in a subject orally or parentally, such as intravenously. More preferably, the Selexipag is administered orally. The term "orally" usually includes the forms of oral tablet, oral capsule, oral liquid, oral disintegrating tablet, sublingual, buccal, oral pellet, oral film, oral gel, or spray, preferably oral tablet.

In some embodiments, the Selexipag is administered orally once daily, twice daily, thrice daily, or forty daily, such as once daily or twice daily.

The Selexipag is administered at an effective dose for treating and/or preventing atherosclerosis. Effective doses of Selexipag may vary depending upon many different factors, including the severity of the disease, means of administration, target site, physiological state of the subject, the medical history of the subject, whether the subject is human or an animal, other medications administered, whether treatment is prophylactic or therapeutic, and the discretion of the physician. Treatment dosages can be titrated to optimize safety and efficacy.

The Selexipag may be administered to a subject at a dose of at least about 10 µg. For example, the Selexipag may be administered to a patient at a dose from 200 µg to 3200 µg per day, such as from 200 µg to 3000 µg per day, such as from 200 µg to 2800 µg per day, such as from 200 µg to 2600 µg per day, such as from 200 µg to 2400 µg per day, such as from 200 µg to 2200 µg per day, such as from 200 µg to 2000 µg per day, such as from 200 µg to 1800 µg per day, such as from 200 µg to 1600 µg per day, such as from 200 µg to 1400 µg per day, such as from 200 µg to 1200 µg per day, such as from 200 µg to 1000 µg per day, such as from 200 µg to 800 µg per day, such as from 200 µg to 600 µg per day, such as from 200 µg to 400 µg per day.

For example, the Selexipag may be administered to a subject at a dose of 200 µg per day, 400 µg per day, 600 µg per day, 800 µg per day, 1000 µg per day, 1200 µg per day, 1400 µg per day, 1600 µg per day, 1800 µg per day, 2000 µg per day, 2200 µg per day, 2400 µg per day, 2600 µg per day, 2800 µg per day, 3000 µg per day, or 3200 µg per day.

In view of the present disclosure, it has to be understood that the present invention encompasses a composition comprising Selexipag, such as a pharmaceutical composition, for use in the treatment and/or the prevention of atherosclerosis in a subject. Therefore, the above specific embodiments also apply to a composition comprising Selexipag for use in the treatment and/or the prevention of atherosclerosis in a subject.

Pharmaceutical compositions containing Selexipag as the active ingredient can be prepared by intimately mixing the compound or compounds with a pharmaceutically acceptable carrier according to conventional pharmaceutical compounding techniques.

The Selexipag may be associated with at least one other compound known to play a role in the treatment and/or prevention of atherosclerosis. The administration may be separate, simultaneous or sequential.

In some embodiments, the Selexipag is associated with a hypocholesterolemic agent, such as statin, ezetimide, a PCSK9 inhibitor, or a combination thereof. An example of combination is the combination of statin/ezetimide.

In some embodiments, the Selexipag is associated with ezetimibe, cholestyramine, lomitapide, ethyl esters of omega-3 acids, antiplatelet agents, anticoagulant agents, anti-inflammatory agents, blood pressure medications, and/or fibrates.

### Examples

### Example 1: Effect of Selexipag on HDL-induced cholesterol efflux

### Materials and methods

### Cell culture

Contrary to what was previously established, recent studies indicate that foam cells-derived from vascular smooth muscle cells (VSMCs) represent the majority of foam cells in advanced atherosclerotic plaques in humans. [16] [17]. Therefore, the tests involved VSMCs.

Primary cultures of aortic smooth muscle cells (AoSMC) obtained from a human diabetic and obese donor (female) were purchased from Lonza (D-AoSMC CloneticsTM). Cells were cultivated on 12-well plates at 50 000 cells/well in a growth medium composed of the Smooth Muscle Basal Medium (SmBM, Lonza) supplemented with 0.2% hEGF, 0.1% insulin, 0.2% hFGF-B, 5% Fetal Bovine Serum (FBS) and 0.1% gentamicin/amphotericin-B (SingleQuots, Lonza, concentrations not specified) according to Lonza's instructions. The foam phenotype was induced when cells reached a subconfluent density. During all the experiments, cells were maintained within an atmosphere enriched with 5% CO2 at 37°C and were used at passages ranging from 6 to 9.

### Induction of the foam phenotype

Twenty-four hours before any treatment, subconfluent cells were rendered quiescent by incubation in a serum and growth factor-free medium enriched with 0.1 % bovine serum albumin (BSA, Merck) and 20 mM Hepes (Life Technologies) (starvation medium). Thereafter, SMC were treated sequentially with Platelet-Derived Growth Factor-BB (PDGF-BB, 10 ng/mL 24h, PeproTech) and oxidized Low-Density Lipoprotein (oxLDL, 75 µg/mL, 96h, Life Technologies).

### MRE-269 treatment

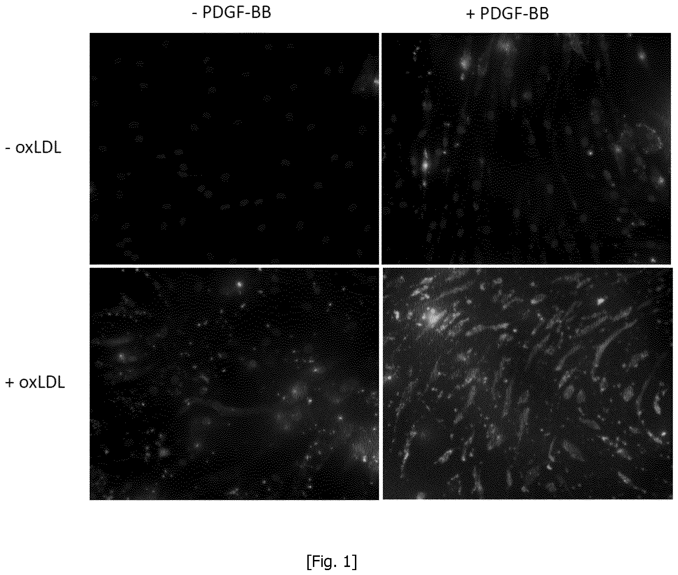

MRE-269 (Cayman Chemical Company) was diluted in DMSO at a concentration of 1 mg/mL, aliquoted in small volumes and stored at -80°C. Every aliquot was used only once. Cells were pre-treated with MRE-269 (100 nM) two hours before adding PDGF-BB and concomitantly with reconstituted High-Density Lipoprotein (rHDL) during cholesterol efflux assays. Regarding **Figure 1****,** VSMCs treated with PDGF-BB and oxLDL accumulate neutral lipids VSMCs were treated sequentially with PDGF-BB (10ng/mL for 24 hours) and oxLDL (75 µg/mL for 96h).

### Radioisotopic cholesterol efflux assay

Cholesterol efflux assay was performed as previously described [7] by using [3H]-cholesterol-loaded VSMCs. Briefly, PDGF-BB-treated VSMCs were incubated with [3H]-cholesterol radiolabeled oxLDL (96h, 75 µg/mL, 1 µCi/mL) in 1 mL of starvation medium. The medium was then replaced by the starvation medium deprived of BSA and cells were treated with MRE-269 (100nM) and/or rHDL prepared as previously described [18] (in the absence or presence of 20 µg/mL rHDL, for 24h). Finally, culture media were harvested and cleared of floating cells and cellular debris by a brief centrifugation. Fractional cholesterol efflux, expressed as a percentage, was calculated as the amount of the radioactivity recovered in the cleared medium divided by the total radioactivity (radioactivity in the medium + radioactivity in the cells). Radioactivity in the cells was obtained after the lipid extraction in a mixture of hexane isopropanol (3:2 v/v) and was determined by liquid scintillation counting on a Wallac Trilux 1450 Microbeta scintillation counter (PerkinElmer, MA, USA). All cholesterol efflux determinations were performed in duplicate for each experiment.

### RNA extraction

Cells were lysed in the "BL" buffer, complemented with 1% thioglycerol (both from the ReliaPrep^{™} RNA Cell Miniprep System, Promega). RNA extraction was then done using the ReliaPrep^{™} RNA Cell Miniprep System (Promega) according to manufacturer's protocol. RNA concentration was measured on a Nanodrop spectrophotometer at a wavelength of 260 nm. The purity of the sample was estimated by calculating the ratio OD260/280 which should be around 2.

### Reverse Transcription

0.5 to 1 µM of RNA were first denaturized for 5 minutes at 68°C in the presence of 1 µM of Oligo-dT (Invitrogen). Reverse transcription mRNA into cDNA was done at 37°C for 60 min in a reactional mix (50 mM Tris-HCl pH 8.3, 75 mM KCl, 3 mM MgCl2, 20 mM dithiothreitol) containing 0.5 mM desoxyribonucleotide triphosphates (dNTP), 200U of RT-MMLV enzyme (Invitrogen) and 20U of RNAse inhibitors (RNAse OUT, Invitrogen). At the end of the reaction, samples were incubated at 70°C for 15 minutes to inactivate the reverse transcription enzyme.

### Quantitative Polymerase Chain Reaction (gPCR)

Quantitative PCR was done using 10 to 20 ng of cDNA, 6 µL of LightCycler 480 SYBR Green I Master (2X) from Roche (which contains all the necessary components for a qPCR, including Taq polymerase, dNTP, salts and SYBR Green) and 1 µL of each primer (forward and reverse, at a concentration of 500nM). qPCR reaction was performed with the LightCycler 480 Thermocycler (Roche). The following program was used: denaturation and activation of the enzyme at 95°C for 5 min, followed by 40 amplification cycles (denaturation at 95°C for 10 sec, hybridization of cDNA with primers at 60°C for 10 sec and extension at 72°C for 10 sec). Each sample of PCR was performed in duplicate. At the end of the reaction, primer specificity was verified by analyzing melting curves on the LightCycler 480 ^{®} Software 1.5. The expression of each target gene was normalized to a housekeeping gene (HSP90), which is an internal control. Sequence primers are listed in **table 1.**

**Table 1: Sequences of the primers used for qPCR**

| Target gene | Forward Primer | Reverse Primer |
|---|---|---|
| ACTA2 | CTATGCCTCTGGACGCACAACT (SEQ ID NO: 1) | CAGATCCAGACGCATGATGGCA (SEQ ID NO: 2) |
| Calponin | CCAACGACCTGTTTGAGAACACC (SEQ ID NO: 3) | ATTTCCGCTCCTGCTTCTCTGC (SEQ ID NO: 4) |
| CD68 | AAAACCAAGGTCCAGGGAAC (SEQ ID NO: 5) | AGGTCCTGCATGAATCCAAA (SEQ ID NO: 6) |
| HSP90 | CAAGTCTGGGACCAAAGCGTTC (SEQ ID NO: 7) | CTTTCTCAGCAACCAAATAAGCAG (SEQ ID NO: 8) |
| IL6 | CAGGAGCCCAGCTATGAACT (SEQ ID NO: 9) | AGCAGGCAACACCAGGAG (SEQ ID NO: 10) |
| Lipa | GTGGGTCATTCTCAAGGCACCA (SEQ ID NO: 11) | CCATAGGGCTAGTACAGAAGGC (SEQ ID NO: 12) |
| SM22 | TCCAGGTCTGGCTGAAGAATGG (SEQ ID NO: 13) | CTGCTCCATCTGCTTGAAGACC (SEQ ID NO: 14) |

### Neutral lipids staining

After foam phenotype induction, SMC were washed 2 times with Dulbecco's phosphate buffered saline (DPBS) and fixed with paraformaldehyde 4% (w/v) during 15 minutes at +4°C before being incubated at room temperature in the dark with Bodipy 493/503 (2µM, ThermoFischer). Then, nuclei were stained with 4'-6'diamidino-2-phenylindol (DAPI) for 5 minutes. Representative fluorescent microscopic images were captured using an inverted Nikon Eclipse-ti (Magnification x20).

### Statistical analysis

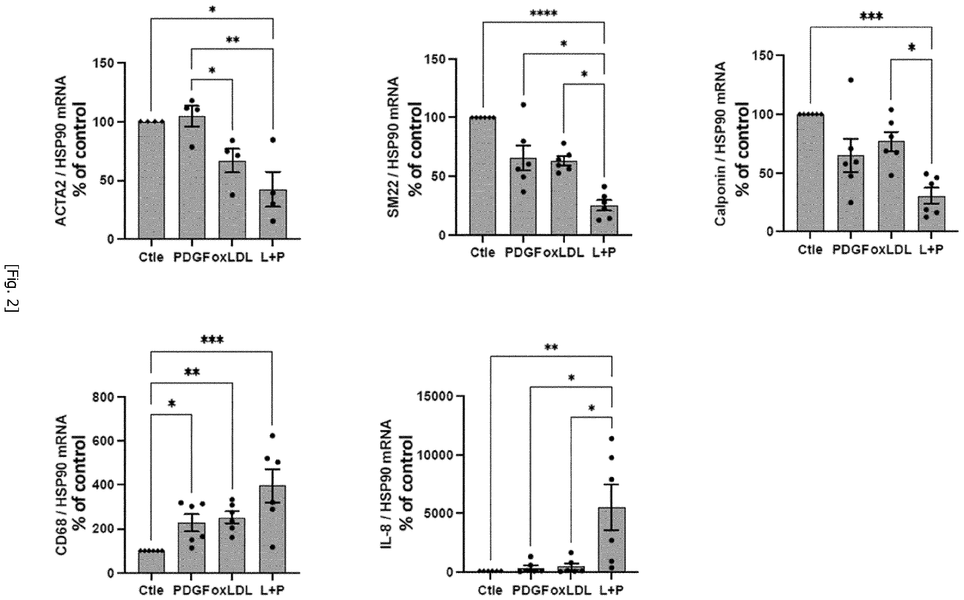

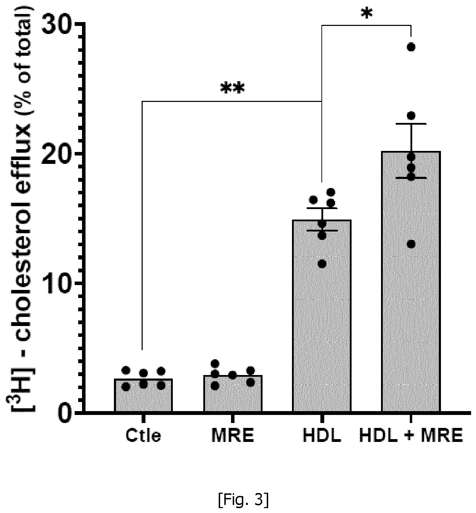

Regarding **Figure 2****,** the results are expressed as the percent (%) of untreated cells. All data are presented as the mean ± SEM of at least 4 independent experiments (n=4 for **Figure 1****,** n=4-6 for **Figure 2****,** n=6 for **Figure 3****).** Non parametric Mann-Whitney tests were performed to compare different experimental conditions. Differences were considered significant if P<0.05.

### Results

In order to obtain a clinically relevant model of VSMC-derived foam cells, we used primary cultures of aortic SMC (AoSMC) derived from an obese and diabetic patient. Before being treated with human oxLDL, cells were incubated with Platelet-derived growth factor-BB (PDGF-BB) during 24 hours. Indeed, PDGF-BB is a growth factor secreted by macrophages at all stages of atherosclerosis. Best known for enabling SMC migration from the arterial media towards the sub-endothelial space where plaques will develop, it could also be involved in the acquisition of a foam and inflammatory phenotype by VSMCs. Indeed, Inaba et al. demonstrated that PDGF-BB markedly increases cholesterol ester incorporation by primary cultures of human umbilical artery SMC treated with acetylated LDL [19] and our group showed that it highly stimulates oxLDL internalization by VSMCs and that it's a potent inducer of the cancer-related transcription factor Slug found in cells co-expressing CD68 (a foam cell and a macrophage marker) and the contractile marker Smooth Muscle Myosin Heavy Chain (SMMHC) all around the lipid necrotic core of human atherosclerotic plaques [20]. Furthermore, PDGF-BB has been shown to be a potent inducer of the KLF4 transcription factor which positively regulates both the acquisition of a "macrophage-like" phenotype by VSMCs and plaque vulnerability in a mouse model of atherosclerosis [21]. Finally, overexpression of the β-isoform of the PDGF receptor (PDGFR) in VSMCs *in vivo* in mice dramatically increases the number of atherosclerotic lesions, plaque vulnerability and the secretion by VSMCs of numerous pro-inflammatory chemokines [22].

PDGF-BB pre-treatment (10 ng/mL) was followed by an incubation with ox-LDL (75 µg/mL) during 4 days corresponding to time required for the appearance of droplet-like structures in cells.

Cellular uptake and metabolism of cholesterol was confirmed by Bodipy staining. As shown in **Figure 1****,** PDGF-BB combined with oxLDL allows a strong accumulation of neutral lipids in nearly all cells. When cells are incubated with PDGF-BB or oxLDL alone, only a few cells harbor neutral lipids (with no apparent differences between both conditions). No lipids are detected in the control.

Since VSMC-derived foam cells are also characterized by a decrease of contractile markers and an increase in macrophage and inflammatory markers, we analyzed by reverse transcriptase-PCR (RT-PCR) gene expression of (i) the SMC contractile markers smooth muscle alpha-actin (SMA, encoded by the *ACTA2* gene), calponin and SM22, (ii) the macrophage marker CD68, and (iii) the interleukin 8 (IL8) inflammatory cytokine. As shown in **Figure 2****,** whereas neither PDGF-BB nor oxLDL alone affect ACTA2, SM22 and calponin mRNA levels compared to the control condition, combination of both results in a strong and significant decrease of all three mRNA levels (respectively -58%, -75% and -69%). In contrast, all experimental conditions allow a significant increase in CD68 gene expression, the most potent being the condition combining PDGF-BB and oxLDL which allows a +400% increase in the CD68 mRNA level relative to the control. Concerning IL8 gene expression, whereas PDGF-BB and oxLDL do not affect it, both allow a strong and significant increase of this gene (+ 5514%).

Indeed, VSMCs treated with PDGF-BB and ox-LDL show typical gene regulations and neutral lipids accumulation characterizing the acquisition of a foam phenotype by these cells.

We therefore investigated in this model the ability of MRE-269 to improve reconstituted HDL-induced cholesterol efflux. Discoidal rHDL are prepared by cholate dialysis from a mixture of apoA-I, isolated from human ethylenediaminetetraacetic acid (EDTA) plasma, and 1-palmitoyl-2-oleoyl-sn-glycero-3-phosphocholine (POPC) 16:0/18:1.

As shown in **Figure 3****,** as attempted, rHDL allow a significant increase in cholesterol efflux (15%) and this effect is significantly potentiated by MRE-269 (20.21 %: majoration of 135% compared to rHDL). Basal cholesterol efflux is about 2.6 % and is not modified by MRE-269 alone.

### Example 2: Effect of Selexipag on internalisation of oxidized LDL (oxLDL) by human aortic Smooth Muscle Cells (AoSMC) in primary culture

### Materials and methods

### Measurement of the incorporation of oxidized LDL (oxLDL) by human aortic Smooth Muscle Cells (AoSMC) in primary culture

The human aortic SMC come from a donor with no known cardiovascular risk factors.

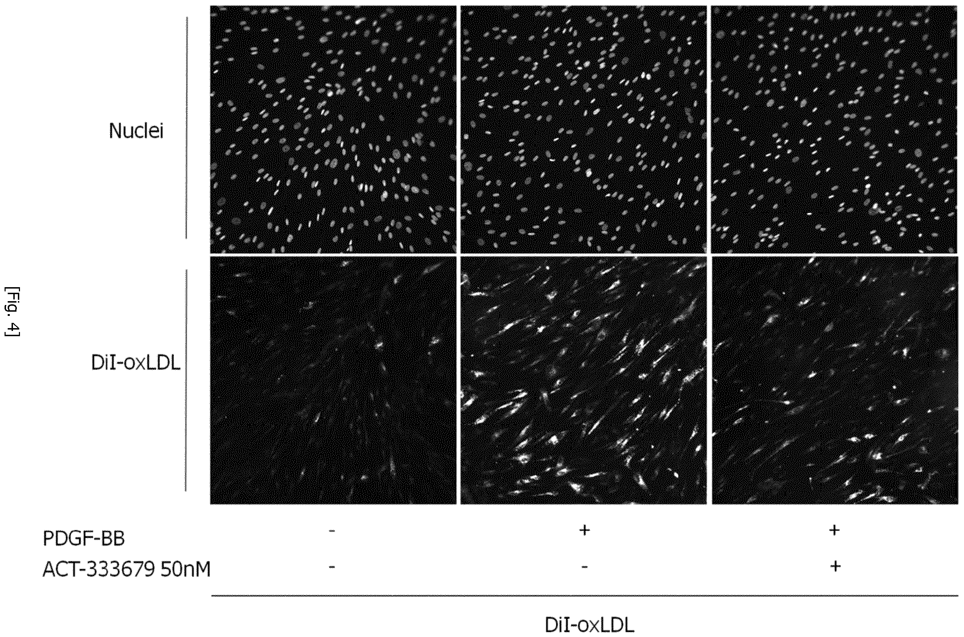

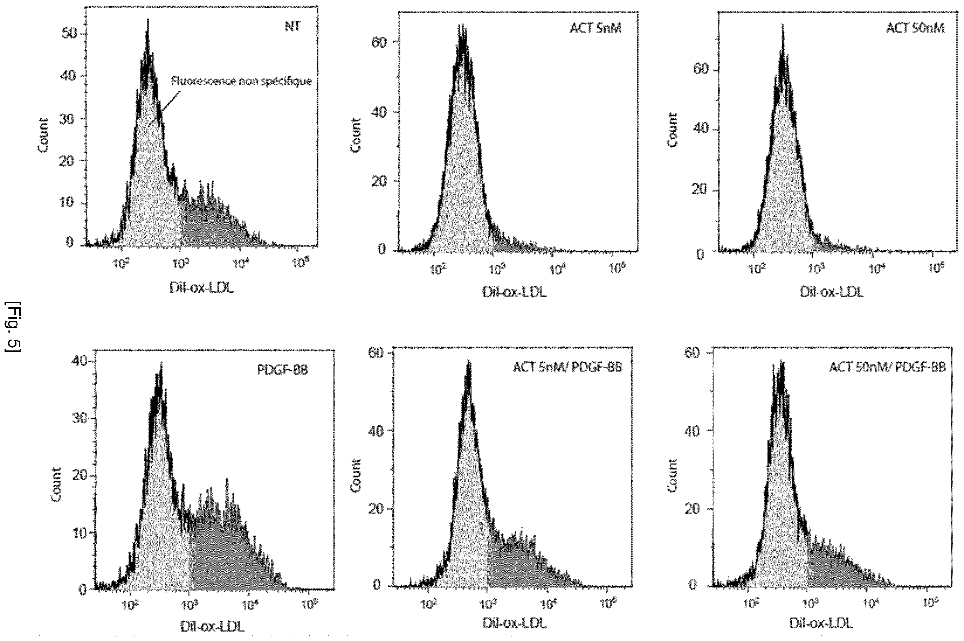

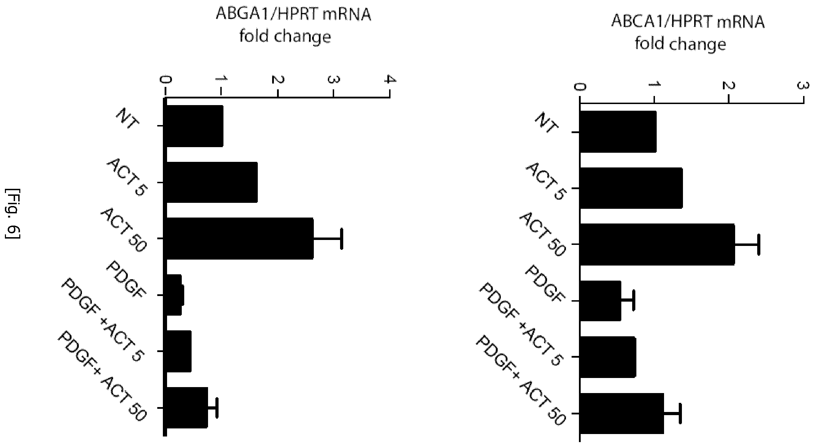

The cells, grown to confluence according to the supplier's instructions (Lonza), were serum-depleted, pre-treated for 1h with increasing concentrations of ACT-333679 (i.e. MRE-269), and incubated in the presence of 10ng/mL PDGF-BB for 24h for **Figures 4** and **5****,** and 6h for **Figure 6****.** The treated cells were then incubated during 4 hours with 10µg/mL of fluorescently labelled DiI-oxLDL **(****Figures 4** and **5****)** and their ability to internalise the oxLDL was analysed by fluorescence microscopy **(****Figure 4****)** and FACS **(****Figure 5****).** FACS allows precise quantification of the number of fluorescent cells (i.e. those that have incorporated fluorescent oxLDL) and the intensity of the incorporated fluorescence per cell (i.e. the quantity of incorporated oxLDL/cell). Nuclei were stained with 4'-6'diamidino-2-phenylindol (DAPI) for analysis by fluorescence microscopy **(****Figure 4****)** or cells were directly analysed by flow cytometry **(****Figure 5****).** A detailed description of the protocol and apparatus used is given in the art [20].

Regarding **Figure 4****,** representative images of the analysis of 5 fields examined in two experiments (n=2). Regarding **Figure 5****,** flow cytometry histograms from a single experiment (n=1) independent of those that generated **Figure 4****.** Regarding **Figure 6****,** the experiment was carried out once (n=1) or twice (n=2), in the latter case the results represent the average of the results obtained for the two experiments.

### Effect of ACT-333679 (MRE-269) on genes encoding transporters involved in cholesterol efflux: ABCA1 and ABCG1

The expression of genes encoding transporters involved in cholesterol efflux (ABCA1: ATP-binding cassette A1 and ABCG1: ATP-binding cassette subfamily G member 1) was measured by real-time RT-PCR. The results are normalized with the gene encoding Hypoxanthine PhosphoRibosyl Transferase (HPRT). The oligonucleotides and technique used are given in the art. [20]

### Results

The acquisition of a foamy phenotype involves the internalisation of oxidized LDL (oxLDL or LDLox) into cells, a phenomenon which is potentiated in the inflammatory context of atherosclerosis, in which PDGF-BB is a major player. The active ingredient of Selexipag (i.e. ACT-333679) inhibits the incorporation of oxLDL, whether basal or stimulated by PDGF-BB, from as low as 5nM **(****Figures 4** and **5****).**

In addition, Selexipag increased basal expression of genes encoding ABCA1 and G1, transporters involved in cholesterol efflux, limiting the foamy phenotype. Expression of these PDGF-BB-inhibited genes was partially restored when the cells were pre-treated with the active ingredient of Selexipag (i.e. ACT-333679) **(****Figure 6****).**

Similar results have been obtained with aortic smooth muscle cells (AoSMC) derived from an obese and diabetic patient, such AoSMC were treated with 100nM of MRE-269 (Data not shown).

### REFERENCES

[1] Lee et al., Cholesterol Efflux Capacity and Its Association With Adverse Cardiovascular Events: A Systematic Review and Meta-Analysis. Front Cardiovasc Med. 2021 Dec 13; 8:774418. doi: 10.3389/fcvm.2021.774418. PMID: 34966797, PMCID: PMC8710716
[2] Kingwell et al., HDL-targeted therapies: progress, failures and future. Nat Rev Drug Discov. 2014 Jun;13(6):445-64. doi: 10.1038/nrd4279. Epub 2014 May 23. PMID: 24854407
[3] Jebari-Benslaiman et al., Cholesterol Efflux Efficiency of Reconstituted HDL Is Affected by Nanoparticle Lipid Composition. Biomedicines 8, no. 10: 373. https://doi.org/10.3390/biomedicines8100373
[4] Kingwell et al., Antiatherosclerotic Effects of CSL112 Mediated by Enhanced Cholesterol Efflux Capacity. Journal of the American Heart Association c. 2022 Apr 19;11(8):e024754. doi: 10.1161/JAHA.121.024754. Epub 2022 Apr 12. Erratum in: J Am Heart Assoc. 2023 Mar 7; 12(5):e027554. PMID: 35411789; PMCID: PMC9238469
[5] Hexing et al., Effects of native and myeloperoxidase-modified apolipoprotein a-I on reverse cholesterol transport and atherosclerosis in mice. Arterioscler Thromb Vasc Biol. 2014 Apr;34(4):779-89. doi: 10.1161/ATVBAHA.113.303044. Epub 2014 Jan 9. PMID: 24407029; PMCID: PMC3966977
[6] Gibson et al., Rationale and design of ApoA-I Event Reducing in Ischemic Syndromes II (AEGIS-II): A phase 3, multicenter, double-blind, randomized, placebo-controlled, parallel-group study to investigate the efficacy and safety of CSL112 in subjects after acute myocardial infarction. Am Heart J. 2021 Jan; 231:121-127. doi: 10.1016/j.ahj.2020.10.052. Epub 2020 Oct 13. PMID: 33065120
[7] Guerin et al., Association of Serum Cholesterol Efflux Capacity With Mortality in Patients With ST-Segment Elevation Myocardial Infarction. J Am Coll Cardiol. 2018 Dec 25;72(25):3259-3269. doi: 10.1016/j.jacc.2018.09.080. PMID: 30573028
[8] Robichaud et al., Autophagy Is Differentially Regulated in Leukocyte and Nonleukocyte Foam Cells During Atherosclerosis. Circ Res. 2022 Mar 18;130(6):831-847. doi: 10.1161/CIRCRESAHA.121.320047. Epub 2022 Feb 9. PMID: 35137605
[9] Dubland et al., Low LAL (Lysosomal Acid Lipase) Expression by Smooth Muscle Cells Relative to Macrophages as a Mechanism for Arterial Foam Cell Formation. Arterioscler Thromb Vasc Biol. 2021 Jun;41(6):e354-e368. doi: 10.1161/ATVBAHA.120.316063. Epub 2021 Apr 1. PMID: 33792344
[10] Versteylen et al., Combined use of exercise electrocardiography, coronary calcium score and cardiac CT angiography for the prediction of major cardiovascular events in patients presenting with stable chest pain. Int J Cardiol. 2013 Jul 15;167(1):121-5. doi: 10.1016/j.ijcard.2011.12.016. Epub 2012 Jan 5. PMID: 22225760
[11] Joshua et al., Quantification of Lipid-Rich Core in Carotid Atherosclerosis Using Magnetic Resonance T2 Mapping, JACC Cardiovasc Imaging. 2017 Jul; 10(7): 747-756
[12] Nissen et al., Intravascular ultrasound: novel pathophysiological insights and current clinical applications. Circulation. 2001 Jan 30;103(4):604-16. doi: 10.1161/01.cir.103.4.604. PMID: 11157729
[13] Nissen et al., Effect of recombinant ApoA-I Milano on coronary atherosclerosis in patients with acute coronary syndromes: a randomized controlled trial. JAMA. 2003 Nov 5;290(17):2292-300. doi: 10.1001/jama.290.17.2292. PMID: 14600188
[14] Kini et al., Changes in plaque lipid content after short-term intensive versus standard statin therapy: the YELLOW trial (Reduction in Yellow Plaque by Aggressive Lipid-Lowering Therapy). Journal Am Coll Cardiol. 2013 Jul 2;62(1):21-9. doi: 10.1016/j.jacc.2013.03.058
[15] Eriksson et al., Stimulation of fecal steroid excretion after infusion of recombinant proapolipoprotein A-I. Potential reverse cholesterol transport in humans. Circulation. 1999 Aug 10; 100(6):594-8. doi: 10.1161/01.cir.100.6.594. PMID: 10441095
[16] Francis et al., The Greatly Under-Represented Role of Smooth Muscle Cells in Atherosclerosis. Curr Atheroscler Rep. 2023 Oct;25(10):741-749. doi: 10.1007/s11883-023-01145-8. Epub 2023 Sep 4. PMID: 37665492; PMCID: PMC10564813
[17] Grootaert et al., Vascular smooth muscle cells in atherosclerosis: time for a re-assessment. Cardiovasc Res. 2021 Sep 28;117(11):2326-2339. doi: 10.1093/cvr/cvab046. PMID: 33576407; PMCID: PMC8479803
[18] Darabi et al., Phosphatidylserine enhances anti-inflammatory effects of reconstituted HDL in macrophages via distinct intracellular pathways, 2022, https://doi.org/10.1096/fj.201800810R
[19] Inaba et al., Platelet-derived growth factor induces c-fms and scavenger receptor genes in vascular smooth muscle cells, 1992, https://doi.org/10.1016/S0021-9258(18)42387-3
[20] Ledard et al., Slug, a cancer-related transcription factor, is involved in vascular smooth muscle cell transdifferentiation induced by platelet-derived growth factor-BB during atherosclerosis, 2020, DOI: 10.1161/JAHA.119.014276
[21] Shankman et al., KLF4-dependent phenotypic modulation of smooth muscle cells has a key role in atherosclerotic plaque pathogenesis, 2015, DOI: 10.1038/nm.3866
[22] He et al., PDGFRbeta signalling regulates local inflammation and synergizes with hypercholesterolaemia to promote atherosclerosis, 2015, DOI: 10.1038/ncomms8770

## Claims

1. Selexipag for use in the treatment and/or the prevention of atherosclerosis in a subject.

2. Selexipag for use according to claim 1, wherein the Selexipag is used to prevent atherosclerotic cardiovascular disease (ASCVD).

3. Selexipag for use according to claim 2, wherein atherosclerotic cardiovascular disease is one or more disease selected from the group consisting of coronary artery disease (CAD), cerebrovascular disease (CVD) and peripheral artery disease (PAD).

4. Selexipag for use according to any of the preceding claims, wherein the Selexipag is used to prevent atherosclerotic plaque formation, to inhibit atherosclerotic plaque development and/or to reduce atherosclerotic plaque.

5. Selexipag for use according to any of the preceding claims, wherein the Selexipag is used to reduce atherosclerotic plaque lipid core.

6. Selexipag for use according to any of the preceding claims, wherein the Selexipag is used to prevent atherosclerotic plaque rupture.

7. Selexipag for use according to any of the preceding claims, wherein the Selexipag is used to decrease the susceptibility of atherosclerotic plaque to rupture.

8. Selexipag for use according to any of the preceding claims, wherein the Selexipag is used to prevent thrombus associated with atherosclerotic plaque rupture.

9. Selexipag for use according to any of the preceding claims, wherein the Selexipag is used to inhibit vascular smooth muscle cells (VSMCs) from internalising low-density lipoprotein-cholesterol (LDL-C).

10. Selexipag for use according to any of the preceding claims, wherein the Selexipag is used to stimulate cholesterol efflux.

11. Selexipag for use according to any of the preceding claims, wherein the subject has diabetes, hypertension, dyslipidemia and/or obesity.

12. Selexipag for use according to any of the preceding claims, wherein the subject has a high or very high risk of developing atherosclerotic cardiovascular disease, said risk is preferably assessed by the Systematic COronary Risk Evaluation (SCORE), the Systematic COronary Risk Evaluation 2 (SCORE2), the Systematic COronary Risk Evaluation-Older Persons (SCORE-OP) the Systematic COronary Risk Evaluation 2-Older Persons (SCORE2-OP), the Framingham risk SCORE, the Globorisk SCORE, the QRISK, the QRISK2, the Reynolds risk score, or the AHA/ACC ASCVD risk estimator plus.

13. Selexipag for use according to any of the preceding claims, wherein the Selexipag is administered orally.

14. Selexipag for use according to any of the preceding claims, wherein the dose of Selexipag is from 200 µg to 3200 µg per day.

15. Selexipag for use according to any of the preceding claims, wherein the Selexipag is associated with a hypocholesterolemic agent, such as statin, ezetimide, a PCSK9 inhibitor, or a combination thereof.
